# EUROPEAN PATENT APPLICATION

(11) **EP 4 710 905 A1**
(43) Date of publication of application: **18.03.2026**
(21) Application number: 24823063.3
(22) Date of filing: 02.04.2024
(51) Int. Cl.: A61F 13/533, A61F 13/49, A61F 13/53, A61F 13/532

(54) **DISPOSABLE DIAPER**

(30) Priority: 16.06.2023 JP 2023099311
(71) Applicant: Daio Paper Corporation, Ehime 799-0492 (JP)
(72) Inventor: FUJIWARA, Yuto, Shikokuchuo-shi, Ehime 799-0113 (JP)
(74) Representative: Meissner Bolte Partnerschaft mbB
(86) International application number: PCT/JP2024/013548
(87) International publication number: WO 2024/257440

(57) **Abstract**

[Problem]

To achieve an improvement in the maintainability of a urine receiving space and conformability in the crotch.

[Solution]

The above problem is solved by the following configuration. An absorbent 56 has a pair of elongated slits 56L extending in a longitudinal direction LD, the slits 56L being provided on one side and the other side in a width direction WD of a crotch portion M, respectively. The absorbent 56 has a center region 56C defined between the one slit 56L and the other slit 56L, and a pair of side regions 56S defined between the slits 56L on both sides in the width direction WD and lateral edges of the absorbent 56. Grooves 53 are provided in the center region 56C and the side regions 56S in a continuous pattern in the width direction WD over 60% or more of a range thereof in the longitudinal direction LD, the grooves 53 being recessed from at least one of a front surface and a back surface of the absorbent 56 to an interior of the absorbent 56 and having a bottom portion 51 compressed in a thickness direction. The center region 56C has, as compared to the side regions 56S, a fixed thickness in the bottom portions 51 of the grooves 53 and a lower area ratio in the bottom portions 51 of the grooves 53.

## Description

### Technical Field

The present invention relates to a disposable diaper designed to improve the maintainability of a urine receiving space (a gap between a front surface of a crotch portion of the disposable diaper and the skin) and conformability in the crotch.

### Background Art

In disposable diapers, it is common to use an absorbent formed by mixing and accumulating pulp fibers and superabsorbent polymer particles. In order to improve shape retention during and after manufacturing, such an absorbent is generally incorporated as an absorption element in which a wrapping sheet made of crepe paper or the like is wound around the absorbent (see, e.g., PTL 1).

While being sandwiched between both legs, the absorption element of a disposable diaper receives multidirectional forces from both sides in the width direction due to leg movements during walking or the like. Therefore, conformability, which allows deformation in accordance with the crotch width, and shape retention, which retains a preferred wearing shape, are required. Of course, in the absorption element of a disposable diaper, securing the absorption capacity in the crotch portion, or the like, is also required.

For example, in the crotch portion of a general disposable diaper, the intermediate portion in the width direction of the crotch portion forms the bottom when worn, and both lateral sides of the intermediate portion rise to form lateral walls, whereby the crotch portion deforms into a substantially U-shaped cross-sectional shape. As a result, a urine receiving space is formed between the front surface of the crotch portion of the disposable diaper and the skin, thereby reducing leakage even in a case in which the single urine volume is large.

In order to facilitate deformation into a substantially U-shaped cross-sectional shape, it has conventionally been performed to form a pair of slits, which penetrate in the thickness direction, along the longitudinal direction on both sides in the width direction of the absorbent in the crotch portion. In this case, the absorbent becomes likely to bend at the slits, with the center region between the pair of slits forming the bottom and side regions located laterally of the respective slits rising to form lateral walls, whereby the crotch portion of the diaper tends to deform into a substantially U-shaped cross-sectional shape, and the rigidity of the lateral walls is improved by the provision of the absorbent (i.e., the urine receiving space becomes less likely to collapse). Thus, the configuration is also provided with the absorption function. However, such a function is lost or significantly reduced when the superabsorbent polymer particles expand due to urine absorption, causing a reduction in the shape retention of the absorbent and the collapse of the slits.

As a method for improving the shape retention of the absorbent, it is known to provide, in the absorption element, grooves each having a bottom portion compressed in the thickness direction in a pattern such as a lattice-shaped pattern (see, e.g., PTL 1). However, providing the grooves by compression leads to an improvement in rigidity. Therefore, if the grooves are provided by compression in a uniformly dense pattern over the entire absorbent in consideration of shape retention after urine absorption, a portion located at the bottom becomes less likely to bend and less likely to deform (reduce conformability) in accordance with the crotch width when the crotch portion deforms into a substantially U-shaped cross-sectional shape.

### Citation List

### Patent Literature

PTL 1: Japanese Patent Application Laid-open No. 2021-000236

### Summary of Invention

### Technical Problem

Therefore, a primary object of the present invention is to achieve an improvement in the maintainability of a urine receiving space and conformability in the crotch, or the like.

### Solution to Problem

The above problem is solved by the following disposable diaper.

### <First Aspect>

A disposable diaper including:
a crotch portion;
a front portion and a back portion extending to a front side and a back side of the crotch portion, respectively; and
an absorption element having an absorbent provided over a range in a longitudinal direction including the crotch portion, and a wrapping sheet wrapping the absorbent, wherein
the absorbent is formed by mixing and accumulating pulp fibers and superabsorbent polymer particles,
the absorbent has a pair of elongated slits extending in the longitudinal direction, the slits being provided on one side and the other side in a width direction of the crotch portion, respectively,
the absorbent has a center region defined between the one slit and the other slit, and a pair of side regions defined between the slits on both sides in the width direction and lateral edges of the absorbent,
grooves are provided in the center region and the side regions in a continuous pattern in the width direction over 60% or more of a range thereof in the longitudinal direction, the grooves being recessed from at least one of a front surface and a back surface of the absorbent to an interior of the absorbent and having a bottom portion compressed in a thickness direction, and
the center region has, as compared to the side regions, a fixed thickness in the bottom portions of the grooves and a lower area ratio in the bottom portions of the grooves, a fixed area ratio in the bottom portions of the grooves and a greater thickness in the bottom portions of the grooves, or a lower area ratio in the bottom portions of the grooves and a greater thickness in the bottom portions of the grooves.

### (Function and Effect)

This disposable diaper improves both the maintainability of a urine receiving space and conformability in the crotch for the following reasons.
(a) The absorbent becomes likely to bend at the slits, with the center region forming the bottom and each side region rising to form the lateral wall. As a result, the crotch portion of the diaper tends to be deformed into a substantially U-shaped cross-sectional shape, and the rigidity of the lateral walls is improved (i.e., the urine receiving space becomes less likely to collapse). Thus, the configuration is also provided with the absorption function.
(b) Both the center region and the side regions are provided with the grooves in a specified pattern, the grooves being recessed from at least one of the front surface and the back surface of the absorption element to the interior of the absorbent and having the bottom portion compressed in the thickness direction. Therefore, the absorbent is likely to retain the shape even after urine absorption, as compared to a case in which the grooves are not provided.
(c) In addition to the above advantages in (a) and (b), the center region has relatively greater flexibility, and the side regions have relatively higher shape retention. Therefore, when the crotch portion of the diaper is worn in a U-shaped cross sectional state, the lateral walls become more highly rigid and the urine receiving space becomes less likely to collapse, while the portion forming the bottom becomes likely to bend and deform in accordance with the crotch width.

### <Second Aspect>

The disposable diaper according to the first aspect, wherein
the center region and the side regions are each regions in which the grooves continuously extend in a lattice-shaped pattern,
a thickness and a width of the bottom portions of the grooves are the same in the center region and the side regions, and
a maximum area of unit frames formed by the grooves in the center region is 1.5 to 4 times a maximum area of unit frames formed by the grooves in the side regions.

### (Function and Effect)

When the grooves recessed from the front surface of the absorbent to the interior thereof and having the bottom portion compressed in the thickness direction are provided in the absorbent in a continuous lattice-shaped pattern, the bottom portions of the grooves, which become highly dense and highly rigid, continuously extend in at least the width direction. Therefore, regions having the grooves exhibit particularly high shape retention at least in the width direction. Furthermore, there is also an advantage that liquid diffusibility in a direction along the grooves is improved. Here, although the shape retention varies depending on the thickness of the bottom portions of the grooves in the absorbent, the degree of the variation is not significant. Furthermore, when the width of the bottom portions of the grooves is increased, the feel of the hard bottom portions of the grooves tends to be easily transmitted to the skin. Accordingly, as in this aspect, it is preferable to vary the areas of the unit frames formed by the grooves while making the thickness and the width of the bottom portions of the grooves the same between the center region and the side regions. As a result, in addition to an improvement in the shape retention, liquid diffusibility in the side regions becomes higher than that in the center region, thereby reducing side leakage.

### <Third Aspect>

The disposable diaper according to the second aspect, wherein
the absorbent is composed of one or a plurality of layers formed by mixing and accumulating pulp fibers and superabsorbent polymer particles,
a basis weight of the pulp fibers in the absorbent is in a range of 80 to 450 g/m²,
a weight ratio of the pulp fibers to the superabsorbent polymer particles in the absorbent is in a range of 20:80 to 80:20,
a maximum thickness of the absorbent is in a range of 1 to 20 mm,
the thickness of the bottom portions is 5 to 40% of the maximum thickness of the absorbent,
the width of the bottom portions is in a range of 1 to 3 mm, and
the area of the unit frames formed by the grooves in the center region is in a range of 150 to 500 mm².

### (Function and Effect)

The dimension of the grooves can be appropriately determined. However, in the absorbent according to this aspect, it is preferable to form the grooves within the scope of this aspect.

### <Fourth Aspect>

The disposable diaper according to the second or third aspect, wherein
the grooves are formed in an oblique lattice-shaped pattern formed by a first portion that is inclined by 40 to 60° clockwise in a plan view with respect to the longitudinal direction, and a second portion that is inclined by 40 to 60° counterclockwise in a plan view with respect to the longitudinal direction.

### (Function and Effect)

The pattern of the grooves can be appropriately determined. However, the oblique lattice-shaped pattern as in this aspect is preferable.

### <Fifth Aspect>

The disposable diaper according to any one of the second to fourth aspects, wherein
a dimension in the width direction of the unit frames in the side regions is 0.5 times or less a minimum dimension in the width direction of the side regions.

### (Function and Effect)

According to the configuration of this aspect, at least one unit frame is present in the side regions. Therefore, even if the position of the lattice-shaped pattern slightly deviates due to manufacturing errors, the configuration is particularly preferable in terms of both rigidity improvement and diffusibility improvement.

### <Sixth Aspect>

The disposable diaper according to the fifth aspect, wherein
a pattern of the grooves in both the side regions is formed only by slit-adjacent frames formed by lateral edges of the slits and the grooves, lateral-edge-adjacent frames formed by lateral edges of the absorbent and the grooves, and the complete unit frames that have a same dimension and a same shape, and
an area of each slit-adjacent frame and an area of each lateral-edge-adjacent frame in both the side regions are smaller than an area of the unit frames.

### (Function and Effect)

According to the configuration of this aspect, when the diaper deforms into a substantially U-shaped cross-sectional shape, the rigidity of both lateral walls becomes particularly high, which is preferable because the urine receiving space becomes less likely to collapse.

### <Seventh Aspect>

The disposable diaper according to any one of the first to sixth aspects, wherein
both the lateral edges of the absorbent extend linearly along the longitudinal direction, and
the pair of slits extends so as to approach the lateral edges of the absorbent from an intermediate position thereof in the longitudinal direction toward the front side and the back side.

### (Function and Effect)

When the slits have the shape as in this aspect, the center region forms the bottom, with each side region rising to form the lateral wall. In a state in which the crotch portion of the diaper deforms into a substantially U-shaped cross-sectional shape, the lateral walls easily fit to the inner sides of the bases of the thighs, and the lateral walls are lowered toward both sides in the longitudinal direction and thus become less likely to bend. As a result, in that vicinity, the urine receiving space becomes particularly less likely to collapse.

### Advantageous Effects of Invention

According to the present invention, the maintainability of a urine receiving space and conformability in the crotch can be improved.

### Brief Description of Drawings

[Fig. 1]

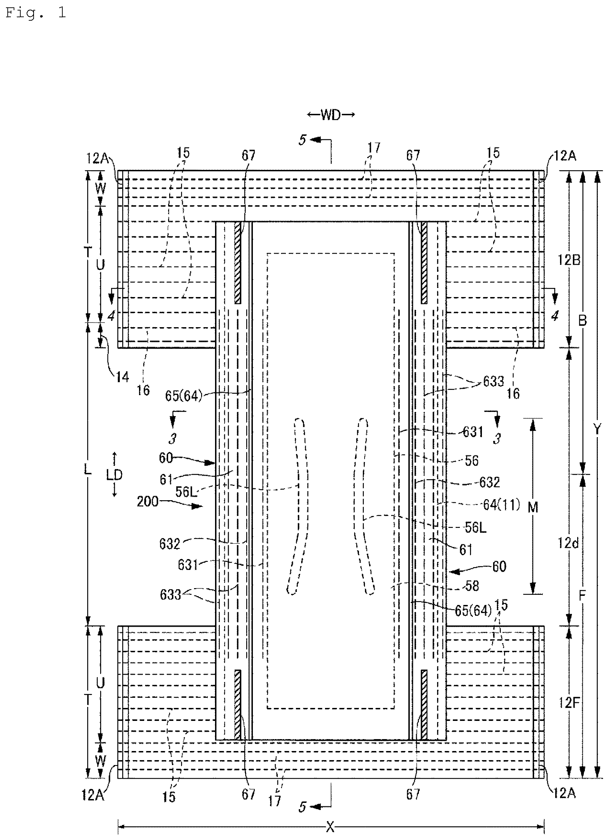

   Fig. 1 is a plan view illustrating the front surface of an underpants-type disposable diaper in a developed state.
[Fig. 2]

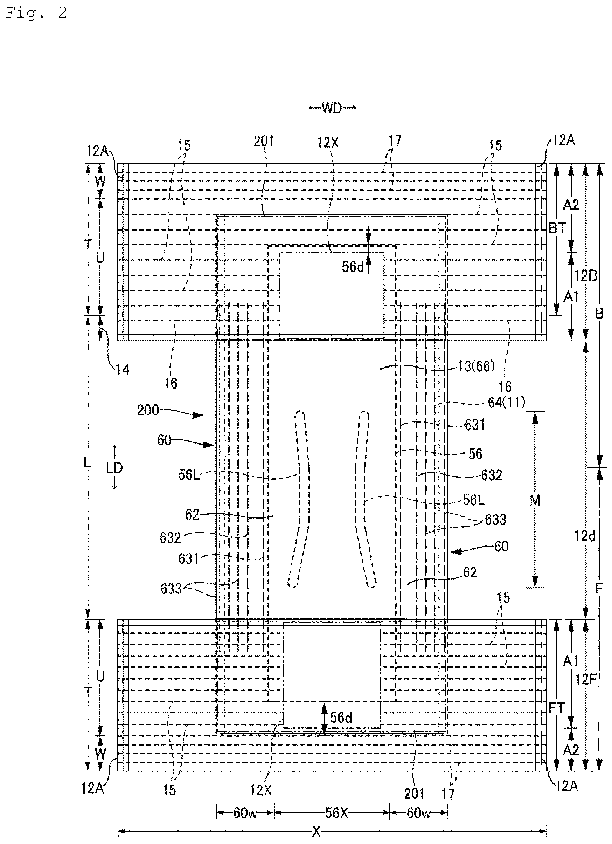

   Fig. 2 is a plan view illustrating the back surface of the underpants-type disposable diaper in a developed state.
[Fig. 3]

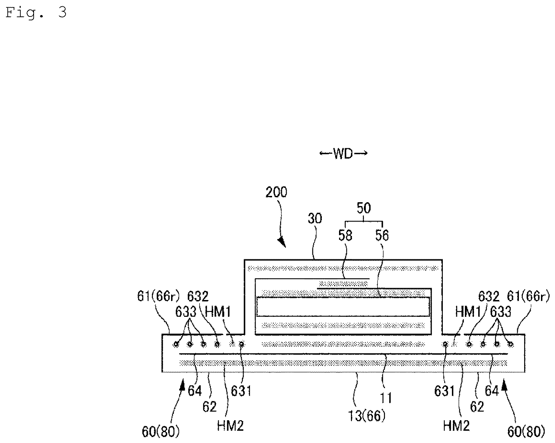

   Fig. 3 is a cross-sectional view taken along line 3-3 in Fig. 1.
[Fig. 4]

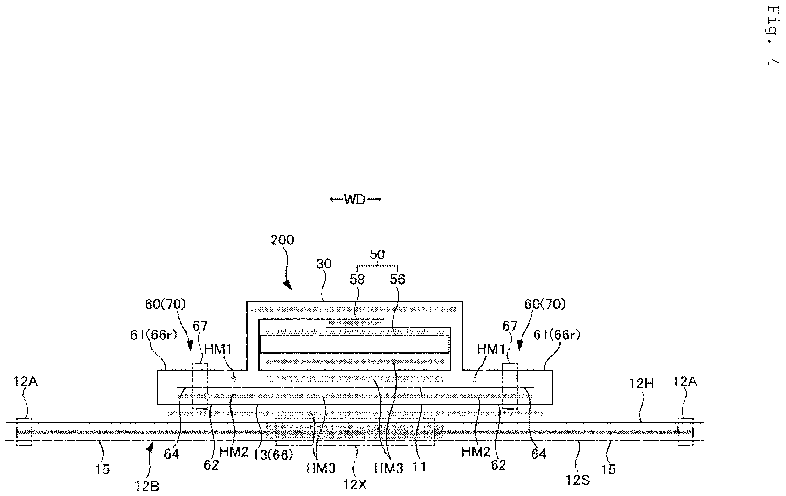

   Fig. 4 is a cross-sectional view taken along line 4-4 in Fig. 1.
[Fig. 5]

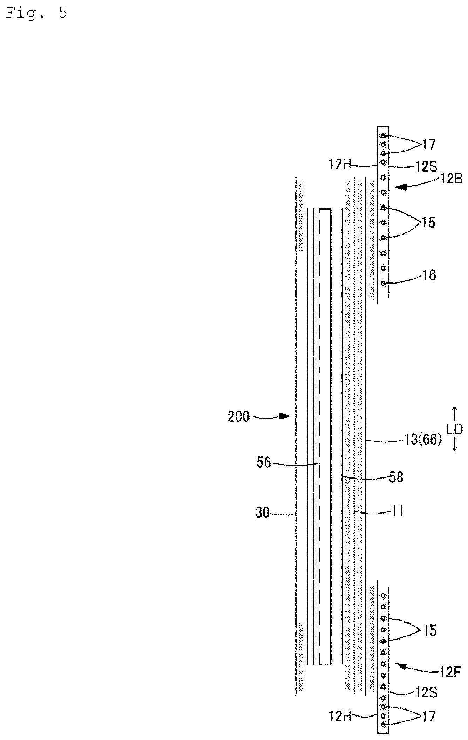

   Fig. 5 is a cross-sectional view taken along line 5-5 in Fig. 1.
[Fig. 6]

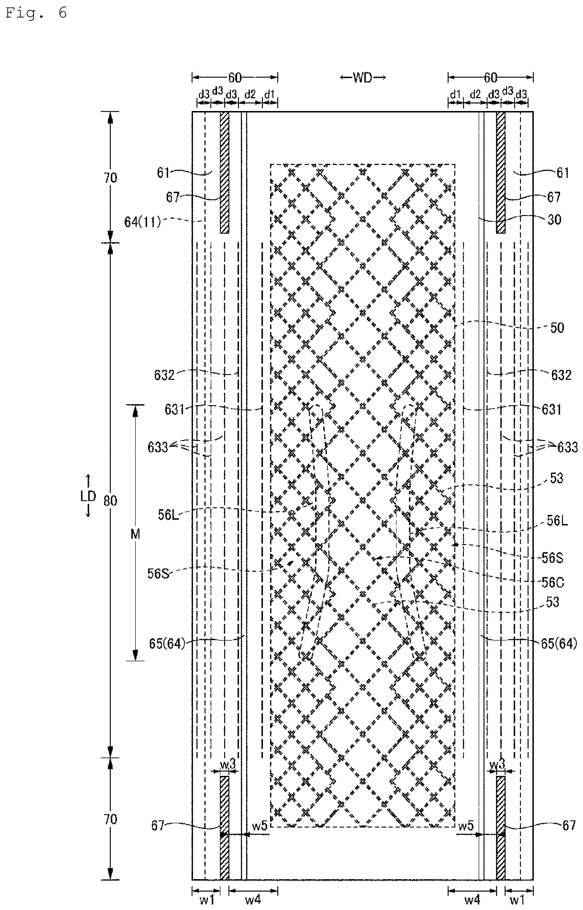

   Fig. 6 is a plan view illustrating the front surface of an interior body.
[Fig. 7]

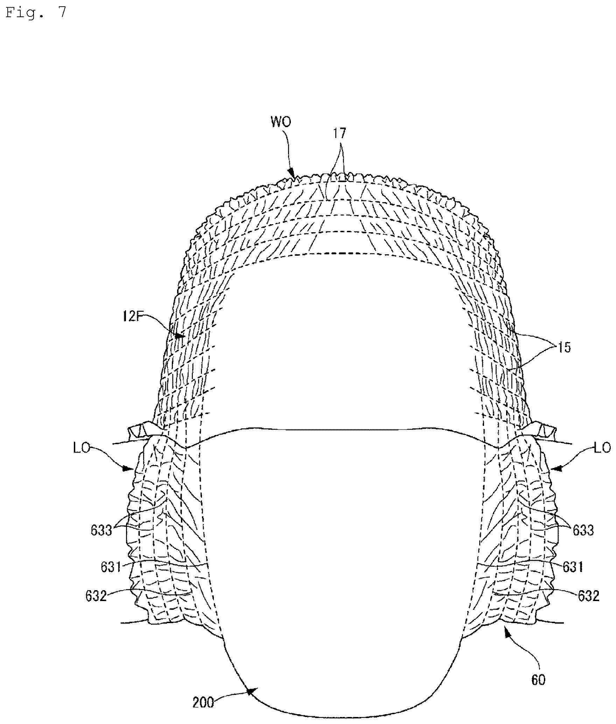

   Fig. 7 is a perspective view of the underpants-type disposable diaper as viewed from the front lower oblique side.
[Fig. 8]

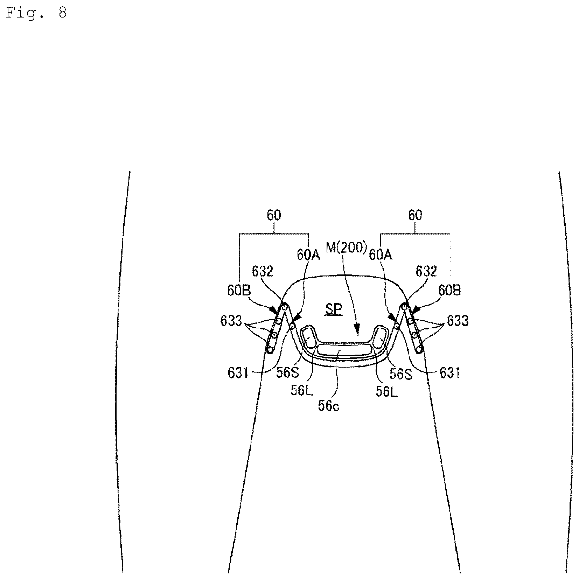

   Fig. 8 is a cross-sectional view of an essential portion in a worn state.
[Fig. 9]

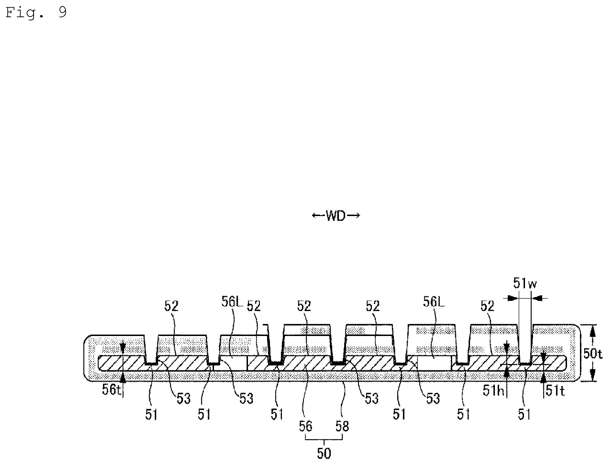

   Fig. 9 is a cross-sectional view of an absorption element.
[Fig. 10]

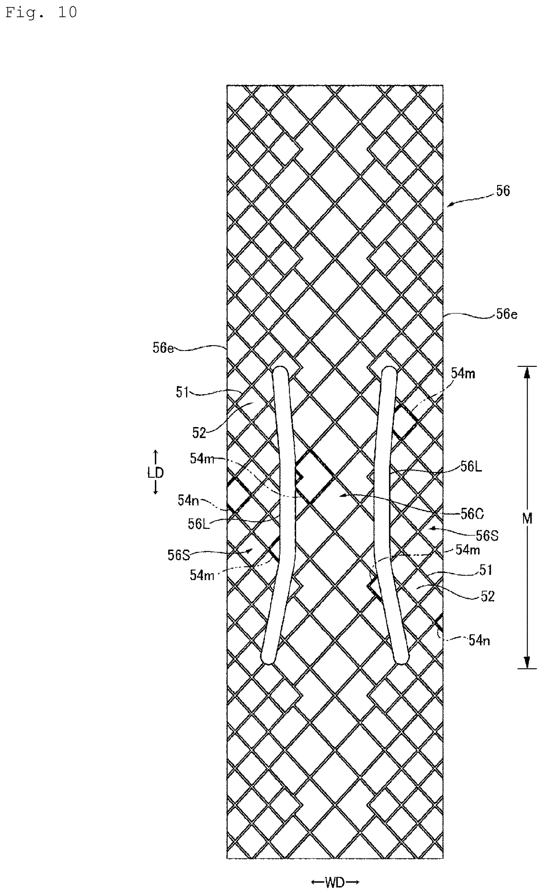

   Fig. 10 is a plan view of an absorbent.
[Fig. 11]

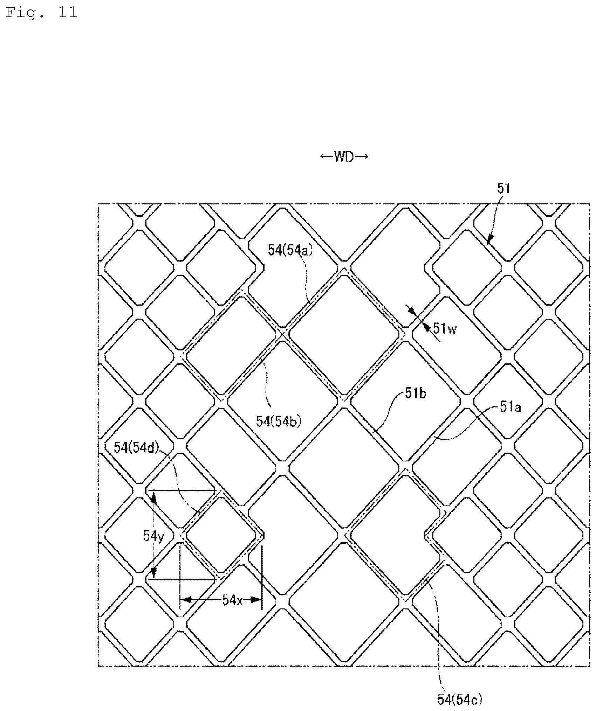

   Fig. 11 is a plan view illustrating the essential portion of Fig. 10 in an enlarged manner.
[Fig. 12]

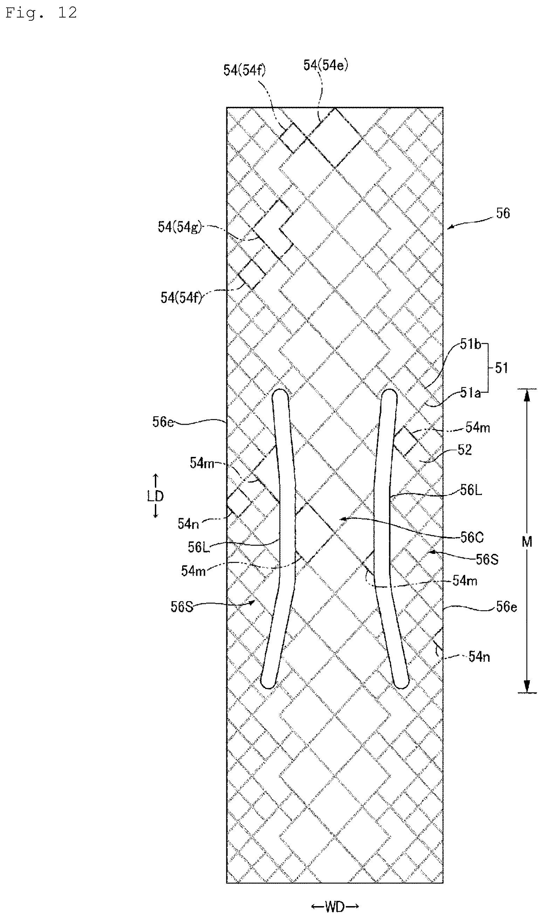

   Fig. 12 is a plan view of the absorbent.
[Fig. 13]

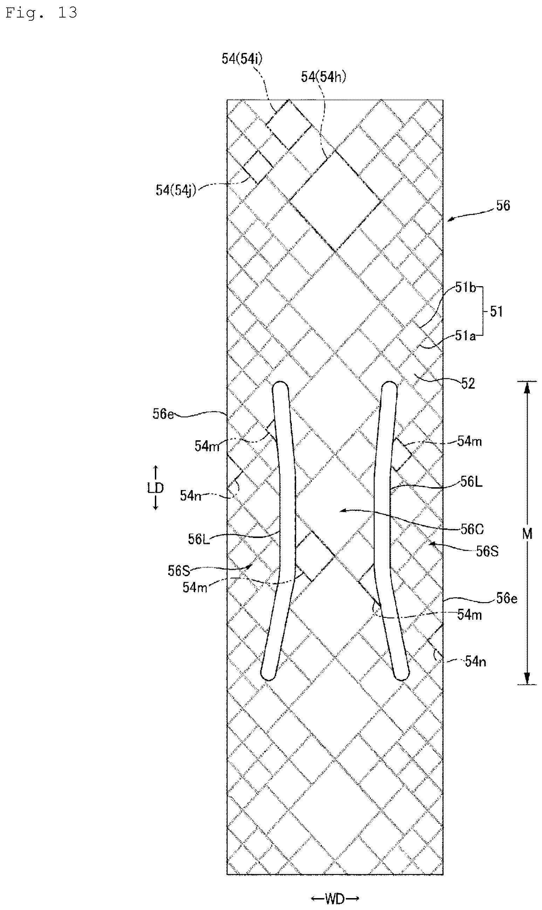

   Fig. 13 is a plan view of the absorbent.
[Fig. 14]

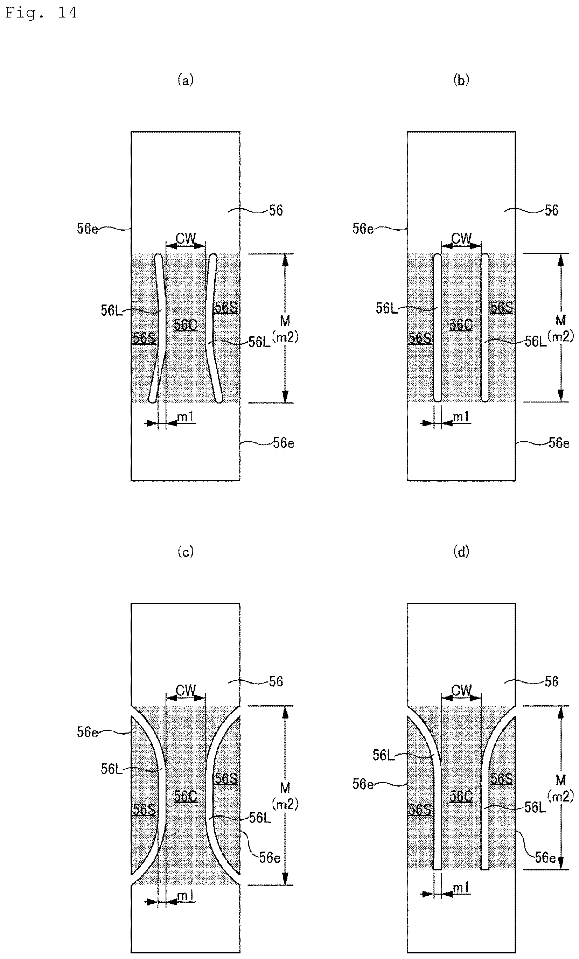

   Fig. 14 is a plan view of the absorbent in the developed state, illustrating an example of slits.

### Description of Embodiments

Hereinafter, the above-described disposable diaper will be described in detail with reference to an example of an underpants-type disposable diaper illustrated in the accompanying drawings. Note that, in each component adjacent in the thickness direction, portions other than the fixed or joined portions described below are also fixed or joined as in known diapers where necessary. In the cross-sectional views, the dotted-pattern portions indicate adhesives, such as hot melt adhesives used as fixing or joining means. The hot melt adhesives can be applied by known methods, such as slot application, continuous linear or dotted bead application, spray application in a spiral shape, a Z-shape, a wavy (regular or irregular) shape, or the like, or pattern coating (transfer of the hot melt adhesives using a relief method). Instead of or together with this, the hot melt adhesives are applied to the outer peripheral surfaces of elastic members at the fixed portions thereof, whereby the elastic members can be fixed to adjacent members. As the hot melt adhesives, there are, for example, an EVA-based type, an adhesive rubber-based type (elastomer-based type), a polyolefin-based type, a polyester/polyamide-based type, and the like, but they can be used without particular limitation. As the fixing or joining means for joining each component, means using material welding, such as heat sealing or ultrasonic sealing, can also be used. In portions where liquid permeability in the thickness direction is required, the components adjacent in the thickness direction are fixed or joined in an intermittent pattern. For example, when such intermittent fixation or joining is performed using the hot melt adhesives, intermittent pattern application in a spiral shape, a Z-shape, a wavy shape, or the like can be preferably used. When application is performed over a range greater than or equal to an application width based on one nozzle, intermittent pattern application in a spiral shape, a Z-shape, a wavy shape, or the like can be performed with or without intervals provided in the width direction. As the joining means for joining each component, means using material welding, such as heat sealing or ultrasonic sealing, can also be used.

Furthermore, as the nonwoven fabric in the following description, known nonwoven fabrics can be appropriately used according to the parts or purposes. As the constituent fibers of the non-woven fabric, for example, synthetic fibers including polyolefin-based fibers such as polyethylene and polypropylene, polyester-based fibers, and polyamide-based fibers (including not only single-component fibers but also composite fibers such as core-sheath fibers), as well as regenerated fibers such as rayon and cupra, and natural fibers such as cotton, can be selected without particular limitation, or these fibers can be used in combination. Crimped fibers are preferably used as the constituent fibers in order to enhance the flexibility of the nonwoven fabric. Furthermore, the constituent fibers of the nonwoven fabric may be hydrophilic fibers (including fibers made hydrophilic by a hydrophilizing agent), hydrophobic fibers, or water-repellent fibers (including fibers made water-repellent by a water-repellent agent). Furthermore, the nonwoven fabric is generally classified according to fiber length, sheet forming methods, fiber joining methods, and lamination structures into staple fiber nonwoven fabric, filament nonwoven fabric, spunbond nonwoven fabric, meltblown nonwoven fabric, spunlace nonwoven fabric, thermal bonded (air-through) nonwoven fabric, needle-punched nonwoven fabric, point-bonded nonwoven fabric, laminated nonwoven fabric (including SMS nonwoven fabric and SMMS nonwoven fabric in which a meltblown layer is interposed between spunbond layers), or the like. However, any of these nonwoven fabrics can be used.

Figs. 1 to 7 illustrate an example of an underpants-type disposable diaper. This underpants-type disposable diaper 100 includes a rectangular front exterior body 12F that constitutes a front lower torso portion FT, a rectangular back exterior body 12B that constitutes a back lower torso portion BT, and an interior body 200 that is joined to intermediate portions in the width direction WD of the exterior bodies 12F and 12B so as to extend from the front exterior body 12F to the back exterior body 12B via a crotch portion M. Both lateral portions of the front exterior body 12F and both lateral portions of the back exterior body 12B are joined to form side seals 12A. As a result, an opening formed by the front edge and the back edge of the exterior bodies 12F and 12B serves as a waist opening WO through which the waist of a wearer passes, and portions, each of which is surrounded by the lower edges of the exterior bodies 12F and 12B and the lateral edge of the interior body 200 on both sides in the width direction WD of the interior body 200, serve as leg openings LO through which the legs pass. The interior body 200 is a portion that absorbs and retains excretions such as urine, and the exterior bodies 12F and 12B are portions designed to support the interior body 200 with respect the body of the wearer. Furthermore, reference numeral Y indicates the overall length of the diaper in the developed state (the length in the longitudinal direction from the edge of the waist opening WO of the front body F to the edge of the waist opening WO of the back body B), and reference sign X indicates the overall width of the diaper in the developed state.

This underpants-type disposable diaper 100 has a lower torso region T that is defined as a longitudinal range having the side seals 12A (a longitudinal range from the waist opening WO to the upper ends of the leg openings LO), and an intermediate region L that is defined as a longitudinal range of the portions forming the leg openings LO (a region between the longitudinal range having the side seals 12A for the front body F and a longitudinal range having the side seals 12A for the back body B). The portions located in lower torso region T of the front exterior body 12F and a back exterior body 12B, that is, a front lower torso portion FT and a back lower torso portion BT, can be conceptually divided into a "waist portion" W that forms the edge portion of the waist opening, and a "lower waist portion" U that is a portion located below the waist portion W. Generally, when the front lower torso portion FT and the back lower torso portion BT internally have a boundary at which the stretching stress in the width direction WD varies (e.g., where the thickness or stretch rate of elastic members vary), the waist portion W is defined as the portion on the waist opening WO side of the boundary closest to the waist opening WO. When no such boundary is present, the waist portion W is defined as a portion extending toward the waist opening WO side from an absorbent 56 or the interior body 200. The longitudinal lengths of the waist portion W and the lower waist portion U vary depending on the size of the product and can be appropriately determined. However, as an example, the waist portion W can be in the range of 15 to 40 mm, and the lower waist portion U can be in the range of 65 to 120 mm. Meanwhile, both lateral edges of the intermediate region L are constricted in a U-shape or a curved shape so as follow the peripheries of the legs of the wearer, and the resulting constricted portions serve as portions into which the legs of the wearer are inserted. As a result, the underpants-type disposable diaper in the developed state has a substantially hourglass shape as a whole.

### (Fixed Regions of Interior Body)

The interior body 200 can be fixed to the exterior bodies 12F and 12B by joining means using material welding, such as heat sealing or ultrasonic sealing, or by using a hot melt adhesive HM3. In the illustrated example, the back surface of the interior body 200 is fixed to the inner surfaces of the exterior bodies 12F and 12B via the hot melt adhesive HM3. In the illustrated example, fixed regions 201 of the interior body 200 are provided in regions where the front lower torso portion FT and the back lower torso portion BT overlap the interior body 200. It is preferable that the fixed regions of the interior body be provided over substantially the entire regions where the exterior bodies 12F and 12B overlap the interior body 200, excluding the peripheral edges of these regions.

### (Exterior Bodies)

As in the illustrated example, the exterior bodies 12F and 12B are composed of the rectangular front exterior body 12F that is the portion constituting at least the front lower torso portion FT (the lower torso portion of the front body F), and the rectangular back exterior body 12B that is the portion constituting at least the back lower torso portion BT (the lower torso portion of the back body B). The exterior bodies 12F and 12B may be configured such that the front exterior body 12F and the back exterior body 12B are not continuous on the crotch side and are spaced apart in the longitudinal direction LD (a two-divided-exterior type). Alternatively, although not illustrated, they may be configured such that the front exterior body 12F and the back exterior body 12B are continuous from the front body F to the back body B via the crotch portion M (an exterior-integrated type). In the exterior-two-divided type, a separation distance 12d in the longitudinal direction can be, for example, approximately 40 to 60% of the overall length Y. In the illustrated example, the lower edges of the front exterior body 12F and the back exterior body 12B have a linear shape along the width direction WD. However, at least one of the lower edges of the front exterior body 12F and the back exterior body 12B may have a curved shape along the peripheries of the legs.

In the underpants-type disposable diaper of the exterior-two-divided type, the interior body 200 is exposed between the front exterior body 12F and the back exterior body 12B. In order to prevent a liquid-impermeable sheet 11 from being exposed at the back surface of the interior body 200, it is preferable that the back surface of the interior body 200 include a cover nonwoven fabric layer 13 that ranges from between the front exterior body 12F and the interior body 200 to between the back exterior body 12B and the interior body 200. The inner surface and the outer surface of the cover nonwoven fabric layer 13 can be adhered to respective facing surfaces via a second hot melt adhesive HM2 and a third hot melt adhesive HM3. The nonwoven fabric used for the cover nonwoven fabric layer 13 can be appropriately selected, for example, from the same material as that of the exterior bodies 12F and 12B.

In the example illustrated in Figs. 1 and 2, the longitudinal dimension of the back exterior body 12B is greater than that of the front exterior body 12F, the front exterior body 12F does not have a portion corresponding to the intermediate region L, and the back exterior body 12B has a gluteal cover portion 14 that extends from the lower torso region T toward the intermediate region L side. However, the dimensions in the longitudinal direction LD of the front exterior body 12F and the back exterior body 12B may be equal, and both the front exterior body 12F and the back exterior body 12B may not have a portion that corresponds to the intermediate region L.

As illustrated in Figs. 3 to 5, the exterior bodies 12F and 12B are formed by joining an outer sheet layer 12S disposed adjacent to the outer sides of elastic members 15 to 17 and an inner sheet layer 12H disposed adjacent to the inner sides of the elastic members 15 to 17, which will be described later, by joining means such as a hot melt adhesive or welding. The outer sheet layer 12S and the inner sheet layer 12H can be formed, as illustrated in Fig. 5, by folding a single sheet material such that a crease is located on the waist opening side, or can be formed by bonding two sheet materials together although not illustrated.

The sheet materials used for the outer sheet layer 12S and the inner sheet layer 12H can be used without particular limitation, but nonwoven fabric is preferable. When nonwoven fabric is used, it is preferable that the basis weight per sheet be in the range of approximately 8 to 20 g/m².

In the exterior bodies 12F and 12B, in order to improve fittability around the lower torso of the wearer, the elastic members 15 to 17 are attached at a specified stretch rate between the outer sheet layer 12S and the inner sheet layer 12H, and an stretchable region is formed that elastically expands and contracts in the width direction WD together with the elastic members 15 to 17. The stretchable region is capable of expanding and contracting between a natural-length state in which the region is contracted together with the elastic members 15 to 17 to form wrinkles or pleats, , and a stretched state in which the region is stretched in the width direction together with the elastic members 15 to 17 up to a specified stretch rate without wrinkles. As the elastic members 15 to 17, not only elongated elastic members (illustrated example) such as rubber threads, but also known elastic members such as strip-shaped, web-shaped, or film-shaped members can be used without particular limitation. The elastic members 15 to 17 may be synthetic rubber or natural rubber. In the exterior bodies 12F and 12B, for bonding the outer sheet layer 12S and the inner sheet layer 12H and for fixing the elongated elastic members 15 to 17 interposed therebetween, at least one of a hot melt adhesive applied by various application methods and fixation means using material welding, such as heat sealing or ultrasonic sealing, can be used. If the entire surfaces of the exterior bodies 12F and 12B are firmly fixed, flexibility is impaired. Therefore, it is preferable that portions other than the adhered portions of the elongated elastic members 15 to 17 be not adhered or weakly adhered. In the illustrated example, a configuration is provided in which a hot melt adhesive is applied only to the outer peripheral surfaces of the elongated elastic members 15 to 17 by application means such as a comb gun or a SureWrap nozzle, so that the elastic members 15 to 17 are interposed between both the sheet layers 12S and 12H, whereby the fixation of the elongated elastic members 15 to 17 to both sheet layers 12S and 12H, as well as the fixation between the sheet layers 12S and 12H, is performed only by the hot melt adhesive applied to the outer peripheral surfaces of the elongated elastic members 15 to 17.

The elastic members 15 to 17 in the illustrated example will be described in further detail. Between the outer sheet layer 12S and the inner sheet layer 12H in the waist portion W of the exterior bodies 12F and 12B, a plurality of waist elastic members 17 are attached at intervals in the longitudinal direction and in an elongated state along the width direction at a specified stretch rate. As a result, a waist stretchable region that is continuous over the overall width direction WD is formed. Furthermore, among the waist elastic members 17, one or more waist elastic members 17 disposed in a region adjacent to the lower waist portion U may overlap the interior body 200, or may be each provided on both sides in the width direction WD of the interior body 200, excluding a central portion in the width direction that overlaps the interior body 200. As the waist elastic members 17, it is preferable to provide approximately 2 to 15 rubber threads, particularly, approximately 4 to 10 rubber threads, which have a thickness of approximately 300 to 940 dtex, particularly, a thickness of approximately 470 to 780 dtex (in the case of synthetic rubber, the thickness is as described above. In the case of natural rubber, it is preferable that the cross-sectional area be in the range of approximately 0.05 to 1.5 mm², particularly, in the range of approximately 0.1 to 1.0 mm²), at intervals of 2 to 12 mm, particularly, at intervals of 3 to 7 mm. Thus, it is preferable that the stretch rate in the width direction WD of the waist portion W be in the range of approximately 150 to 300%, particularly, in the range of approximately 200 to 280%. Furthermore, in the waist portion W, it is not necessary to provide elastic members having the same thickness or the same stretch rate over the entire longitudinal direction LD. For example, it may be possible to provide elastic members that partially differ in thickness or stretch rate.

Furthermore, between the outer sheet layer 12S and the inner sheet layer 12H in the lower waist portion U of the exterior bodies 12F and 12B, a plurality of lower waist elastic members 15, which are made of elongated elastic members, are attached at intervals in the longitudinal direction and in a stretched state along the width direction at a specified stretch rate. As a result, a lower waist stretchable region is formed on both sides in the width direction WD of the interior body 200, excluding a central portion in the width direction that overlaps the interior body 200. As the lower waist elastic members 15, it is preferable to provide approximately 5 to 30 rubber threads, which have a thickness of approximately 300 to 940 dtex, particularly, a thickness of approximately 470 to 780 dtex (in the case of synthetic rubber, the thickness is as described above. In the case of natural rubber, it is preferable that the cross-sectional area be in the range of approximately 0.05 to 1.5 mm², particularly, in the range of approximately 0.1 to 1.0 mm²), at intervals of 1 to 15 mm, particularly, at intervals of 3 to 8 mm. Thus, it is preferable that the stretch rate in the width direction WD of the lower waist portion U be in the range of approximately 200 to 350%, particularly, in the range of approximately 240 to 300%. Furthermore, in the lower waist portion U, it is not necessary to provide elastic members having the same thickness or the same stretch rate over the entire longitudinal direction LD. It may be possible to provide elastic members that partially differ in thickness or stretch rate.

Furthermore, between the outer sheet layer 12S and the inner sheet layer 12H in the gluteal cover portion 14 of the back exterior body 12B, one or a plurality of gluteal cover elastic members 16, which are made of elongated elastic members, are attached at intervals in the longitudinal direction and in a stretched state along the width direction at a specified stretch rate on both sides in the width direction WD of the interior body 200, excluding a central portion in the width direction that overlaps the interior body 200. As a result, a gluteal cover stretchable region is formed on both sides in the width direction WD of the interior body 200, excluding the central portion in the width direction that overlaps the interior body 200. As the gluteal cover elastic members 16, it is preferable to fix approximately 2 to 10 rubber threads, which have a thickness of approximately 300 to 940 dtex, particularly, a thickness of approximately 470 to 780 dtex (in the case of synthetic rubber, the thickness is as described above. In the case of natural rubber, it is preferable that the cross-sectional area be in the range of approximately 0.05 to 1.5 mm², particularly, in the range of approximately 0.1 to 1.0 mm²), at intervals of 5 to 40 mm, particularly, at intervals of 5 to 20 mm, and at a stretch rate of 150 to 300%, particularly, at a stretch rate of 180 to 260%.

Within the front lower torso portion FT and the back lower torso portion BT, it is preferable that a first region, which is composed of a portion that overlaps the absorbent 56 and portions located between this overlapping portion and the side seals 12A, be an intermittent stretchable area A1 that has a non-stretchable region 12X provided at least at an intermediate portion in the width direction WD in the portion that overlaps the absorbent 56, and stretchable regions provided between the non-stretchable region 12X and the side seals 12A. Furthermore, it is preferable that a second region, which is located between the intermittent stretchable area A1 and the waist opening WO, be a continuous stretchable area A2 in which the stretchable region is continuous in the width direction WD from one side seal 12A to the other side seal 12A.

In the illustrated example, the intermittent stretchable area A1 may extend up to the waist opening WO side beyond the range of the first region (beyond the absorbent 56), as long as they include the first region. In the illustrated example, in the back lower torso portion BT, only the first region serves as the intermittent stretchable area A1, and the portion located between the first region and the waist opening WO serves as the continuous stretchable area A2. In the front lower torso portion FT, the region (region extending up to the waist opening WO side beyond the first region), which is composed of the portion that overlaps the fixed region 201 of the interior body 200 and the portions located between this overlapping portion and the side seals 12A, serves as the intermittent stretchable area A1, but is not limited thereto. Accordingly, the arrangements may be reversed. The region, which is composed of the portion that overlaps the fixed region 201 of the interior body 200 and the portions located between this overlapping portion and the side seals 12A, may serve as the intermittent stretchable area A1 in both the front lower torso portion FT and the back lower torso portion BT, or only the first region may serve as the intermittent stretchable area A1 in both the front lower torso portion FT and the back lower torso portion BT. Furthermore, in the illustrated example, the intermittent stretchable area A1 is formed in a range in the longitudinal direction LD that includes only the lower waist stretchable region having the lower waist elastic members 15, but the range may be extended toward the waist opening WO side so as to include a part of the waist stretchable region having the waist elastic members 17.

In the illustrated example, the continuous stretchable area A2 is formed only by the waist stretchable region having the waist elastic members 17 in the front lower torso portion FT, and is formed by both the entire waist stretchable region having the waist elastic members 17 and a part of the lower waist stretchable region including the lower waist elastic members 15 in the back lower torso portion BT. However, the configuration is not limited thereto. Accordingly, the arrangements may be reversed. The continuous stretchable area A2 in both the front lower torso portion FT and the back lower torso portion BT may be formed only by the waist stretchable region having the waist elastic members 17, or may be formed by both the entire waist stretchable region having the waist elastic members 17 and a part of the lower waist stretchable region having the lower waist elastic members 15.

As illustrated in the figures, when the first region, which is composed of the portion that overlaps the absorbent 56 and the portions located between this overlapping portion and the side seals 12A, serves as the intermittent stretchable area A1 rather than the continuous stretchable area A2, the absorbent 56 does not contract in the width direction WD more than necessary, thereby preventing a reduction in fittability, an undesirable bulky appearance, or a decrease in absorption performance. Such an intermittent stretchable area A1 includes not only an area in which the elastic members 15 and 16 are present only on both sides in the width direction WD, but also an area in which the elastic members 15 and 16 are present from one side to the other side in the width direction WD across the absorbent 56, but the intermediate portion or the entire portion in the width direction of the portion that overlaps the absorbent 56 hardly exhibits its contraction force due to fine cutting of the elastic members 15 and 16, or the like (substantially equivalent to a case in which the elastic members 15 and 16 are not provided). In this case, the portion serves as a non-stretchable region 12X, while both sides in the width direction WD serve as stretchable regions (contraction force exhibiting portions). The non-stretchable region 12X is preferably located between both lateral edges of the interior body 200 and more preferably located between both lateral edges of the absorbent 56. The dimension in the width direction WD of the non-stretchable region 12X can be appropriately determined but is preferably in the range of 0.6 to 1.2 times and more preferably in the range of 0.8 to 1.0 times the maximum width of the absorbent 56. Furthermore, the dimension in the width direction WD of the non-stretchable region 12X is preferably in the range of 0.5 to 1.0 times and more preferably in the range of 0.6 to 0.8 times the dimension in the width direction WD of the continuous stretchable area A2.

### (Interior Body)

The shape of the interior body 200 is optional, but it is rectangular in the illustrated example. As illustrated in Figs. 3 to 5, the interior body 200 includes a liquid-permeable top sheet 30 located on the skin side of the wearer, the liquid-impermeable sheet 11, and an absorption element 50 interposed therebetween. Reference numeral 60 indicates a side flap 60 having a planar gather.

### (Top Sheet)

The top sheet 30 can be used without particular limitation as long as it is a liquid-permeable material, such as perforated or unperforated nonwoven fabric, or a perforated plastic sheet. However, as in the example illustrated in Figs. 3 and 4, when the top sheet 30 also serves as a covering material for a liquid-impermeable sheet layer 64, it is preferably nonwoven fabric.

Both lateral portions of the top sheet 30 may be folded back toward the back side of the absorption element 50 at the lateral edges thereof, or may laterally protrude from the lateral edges of the absorption element 50 without being folded back. Furthermore, where necessary, a configuration may be employed in which the overall width of the top sheet 30 is made shorter than the overall width of the absorption element 50 so that both lateral edges of the top sheet 30 are located on the absorption element 50.

### (Intermediate Sheet)

Although not illustrated in the figures, an intermediate sheet (also referred to as a "second sheet") having a liquid permeability rate faster than that of the top sheet 30 can be provided in order to promptly transfer liquid that has passed through the top sheet 30 to the absorbent. This intermediate sheet is designed to improve the absorption performance of the absorbent by promptly transferring liquid to the absorbent and prevent the phenomenon of "returning" of the absorbed liquid from the absorbent. The intermediate sheet can be omitted.

### (Liquid-Impermeable Sheet)

The material of the liquid-impermeable sheet 11 provided on the back side of the absorbent 56 is not particularly limited, but examples of the material include plastic films made of polyolefin-based resins such as polyethylene or polypropylene. In the liquid-impermeable sheet 11, it is preferable to use materials having liquid impermeability and moisture permeability, which have been preferred in recent years from the viewpoint of moisture control. As plastic films having moisture permeability, microporous plastic films obtained by kneading an inorganic filler into polyolefin-based resins such as polyethylene or polypropylene, molding the mixture into a sheet, and stretching the molded sheet in a uniaxial or biaxial direction have been widely used.

As in the example illustrated in Figs. 3 and 4, the liquid-impermeable sheet 11 can be configured to extend into the side flaps to improve the waterproof properties of the planar gathers. Alternatively, although not illustrated in the figures, the liquid-impermeable sheet 11 can have a width that falls within the back side of the absorption element 50, or can be folded back toward the front side at the lateral edges of the absorption element 50 and extended to a location between the absorption element 50 and the top sheet 30.

### (Absorption Element)

As illustrated in Figs. 3, 4, and 9, the absorption element 50 includes the absorbent 56 and a wrapping sheet 58 that wraps the entire absorbent 56. The wrapping sheet 58 can be omitted.

### (Absorbent)

The absorbent 56 is formed by mixing and accumulating pulp fibers and superabsorbent polymer particles. The basis weight of the pulp fibers can be, for example, in the range of approximately 80 to 450 g/m² and more preferably in the range of approximately 80 to 200 g/m².

The superabsorbent polymer particles are mixed into the fiber aggregate. The superabsorbent polymer particles include not only "particles" but also "powder." As the superabsorbent polymer particles, those used in such disposable diapers can be used as they are. For example, it is preferable to use particles in which the proportion of particles that remain on a 500 µm standard sieve (JIS Z8801-1: 2006) after sieving (shaking for 5 minutes) is 30% by weight or less. Furthermore, it is preferable to use particles in which the proportion of particles that remain on a 180 µm standard sieve (JIS Z8801-1: 2006) after sieving (shaking for 5 minutes) is 60% by weight or more.

The material of the superabsorbent polymer particles is not particularly limited, but those having a water absorption capacity of 40 g/g or more (as measured in accordance with JIS K7223-1996, "Testing Method for Water Absorption Capacity of Superabsorbent Polymers") are preferable. Examples of the superabsorbent polymer particles include starch-based particles, cellulose-based particles, and synthetic polymer-based particles. Starch-acrylic acid (salt) graft copolymers, saponified products of starch-acrylonitrile copolymers, crosslinked products of sodium carboxymethyl cellulose, and acrylic acid (salt) polymers, and the like can be used. The shape of the superabsorbent polymer particles is preferably a generally-used powdery form, but other shapes can also be used.

As the superabsorbent polymer particles, it is preferable to use those having a water absorption rate of 70 seconds or less and particularly those having a water absorption rate of 40 seconds or less. If the water absorption rate is excessively slow, a phenomenon so-called returning, in which liquid supplied into the absorbent 56 flows back to the outside of the absorbent 56, is likely to occur.

Furthermore, as the superabsorbent polymer particles, it is preferable to use those having a gel strength of 1000 Pa or more. As a result, even when the absorbent 56 is bulky, the tacky feeling after liquid absorption can be effectively suppressed.

The basis weight of the superabsorbent polymer particles can be appropriately determined according to the required absorption capacity for the absorbent 56. Accordingly, although the basis weight cannot be uniformly specified, it can be in the range of 50 to 350 g/m². When the basis weight of the polymer is less than 50 g/m², it becomes difficult to achieve the absorption capacity. When the basis weight exceeds 350 g/m², the effect reaches saturation.

The ratio of the fibers to the superabsorbent polymer particles in the absorbent 56 is not particularly limited. For example, the weight ratio of the pulp fibers to the superabsorbent polymer particles can preferably be in the range of 20:80 to 80:20, and more preferably in the range of 40:60 to 60:40. Particularly, when the weight ratio of the pulp fibers to the superabsorbent polymer particles is in the range of 40:60 to 60:40, the absorbent 56 can be made thinner when compared to an absorbent having the same area and the same absorption capacity. A thickness 56t of the absorbent 56 is not particularly limited. However, it can preferably be in the range of 1 to 20 mm and more preferably in the range of 2 to 5 mm.

The absorbent 56 is provided over a range in the longitudinal direction LD including the crotch portion M. It is preferable that the absorbent 56 continuously extend from the front lower torso portion FT to the back lower torso portion BT. Within the absorbent 56, it is preferable that the portions located in the fixed regions 201 of the interior body 200 be fixed to the exterior bodies 12F and 12B via the wrapping sheet 58, the liquid-impermeable sheet 11, the hot melt adhesive HM3, and the like. Note that reference numeral 56X indicates the maximum width (overall width) of the absorbent 56.

It is preferable that the absorbent 56 have a substantially rectangular shape as in the illustrated example because it allows the absorption capacity in the crotch portion M to be easily secured. However, the crotch portion M may also have narrowest portions (that is, the absorbent 56 has a minimum width portion located at an intermediate position in the longitudinal direction LD, and widened portions that gradually increase in width from the minimum width portion toward a maximum width portion located on the front side of the crotch portion M and toward a maximum width portion located on the back side of the crotch portion M, respectively).

Note that the crotch portion M refers to a range in the longitudinal direction LD that has slits, which will be described later, and a front portion and a back portion extend to the front side and the back side of the crotch portion M, respectively.

### (Slits)

The absorbent 56 has a pair of elongated slits 56L extending in the longitudinal direction LD, the slits 56L being provided on one side and the other side in the width direction WD of the crotch portion M, respectively. By providing such slits 56L in the absorbent 56, bending of the absorbent 56 along the slits 56L is promoted, thereby making it possible to deform the crotch portion M of the diaper into a substantially U-shaped cross-sectional shape when worn as illustrated in Fig. 8. That is, a center region 56C defined between one slit 56L and the other slit 56L forms the bottom, and a pair of side regions 56S defined between the slits 56L on both sides in the width direction WD and lateral edges 56e of the absorbent 56 rises to form the lateral walls. As a result, the crotch portion M of the diaper deforms into a substantially U-shaped cross-sectional shape. Note that the center region 56C and the pair of side regions 56S are indicated by a dot pattern in Fig. 14.

As long as the slits 56L have an elongated shape extending in the longitudinal direction LD, the slits 56L may extend linearly along the longitudinal direction LD as illustrated in Fig. 14(b), or may extend, as illustrated in Fig. 14(a) and the like, so as to approach the lateral edges of the absorbent 56 from an intermediate position in the longitudinal direction LD of the slit 56L in the longitudinal direction LD toward the front side and the back side, respectively. Furthermore, at least one of the front and back ends of the slits 56L may be spaced apart from the lateral edges of the absorbent 56 as illustrated in Figs. 14(a) and 14(b), or one or both of the front and back terminal ends thereof may reach the lateral edges of the absorbent 56 as illustrated in Figs. 14(c) and 14(d). Furthermore, the front and back ends of the slits 56L can have an appropriate shape. For example, the front and back ends of the slits 56L can have a linear shape as in the example illustrated in Fig. 14(d), can have a shape expanded in a curved form (such as a semicircular arc shape) as in the example illustrated in Fig. 14(a) or the like, or can have a shape in which the corners of both ends are rounded and the intermediate portion is linear although not illustrated in the figures. A width m1 of the slits 56L can be appropriately determined, and can, for example, be in the range of 4 to 12 mm. The width m1 of the slits 56L can be uniform in the length direction or can vary in the length direction. The dimension and arrangement in the longitudinal direction LD of the slits 56L may be appropriately determined. For example, a dimension m2 in the longitudinal direction LD of the slits 56L (equivalent to the dimension in the longitudinal direction LD of the crotch portion M) may be in the range of 200 to 600% of the minimum interval CW between both slits 56L and more preferably in the range of 300 to 500%.

### (Wrapping Sheet)

When the wrapping sheet 58 is used, a liquid-permeable material, such as tissue paper, particularly crepe paper, nonwoven fabric, poly-laminated nonwoven fabric, perforated sheet, and the like, can be used as the material. However, it is preferable to use a sheet that does not allow superabsorbent polymer particles to pass through. When using nonwoven fabric instead of crepe paper, it is preferable to use a hydrophilic nonwoven fabric having dense layers. As such a nonwoven fabric, it is particularly preferable to use a laminated nonwoven fabric (including an SMS nonwoven fabric, an SSMMS nonwoven fabric, and the like) in which one or a plurality of meltblown layers and one or a plurality of protection layers, such as spunbond layers that protect the meltblown layer(s), are laminated. The material can include polypropylene, polyethylene/polypropylene composite materials, or the like. The basis weight of the wrapping sheet 58 can be, for example, in the range of 5 to 40 g/m² and preferably in the range of 8 to 20 g/m².

The wrapping structure of the wrapping sheet 58 can be appropriately determined. However, from the viewpoint of ease of manufacturing or prevention of leakage of superabsorbent polymer particles from the front and back end edges, it is preferable to use the wrapping sheet 58 that is wound in a cylindrical shape so as to surround the front surface, the back surface, and both lateral surfaces of the absorbent 56. The wrapping sheet 58 may protrude from the front edge and the back edge of the absorbent. Alternatively, as in the illustrated example, the front edge and the back edge of the wrapping sheet 58 may substantially align with the front edge and the back edge of the absorbent, respectively.

### (Side Flaps)

As illustrated in Figs. 1 to 4 and Figs. 7 and 8, the interior body 200 preferably has the side flaps 60. The side flaps 60 are a pair of portions extending from the lateral edges of the absorbent 56 toward both sides in the width direction WD. Each of the side flaps 60 extends in the longitudinal direction LD from a region overlapping the lower torso stretchable region of the front body F (a region having the lower waist elastic members 15 in the illustrated example), to a region overlapping the lower torso stretchable region of the back body B (a region having the lower waist elastic members 15 in the illustrated example). Furthermore, the side flaps 60 has a side nonwoven fabric 66 that ranges from the front surface to the back surface of the side flaps 60 via the lateral edge thereof, and elongated gather elastic members 631 to 633 that are provided along the longitudinal direction LD between a front surface portion 61 and a back surface portion 62. The front surface portion 61 and the back surface portion 62 extend over the entire region in the longitudinal direction LD of the side flaps 60. In the illustrated example, the front surface portion 61 is formed by a folded-back portion 66r of the side nonwoven fabric 66 but is not limited thereto. The front end portion and the back end portion of the side flaps 60 do not contract in the longitudinal direction LD together with the gather elastic members 631 to 633, and form a side non-stretchable region 70 having a fixed portion 67 where the front surface portion 61 is fixed to the back surface portion 62. The portion between the longitudinal side non-stretchable regions 70 in the side flaps 60 serves as a plane gather region 80 in which the gather elastic members 631 to 633 are fixed, and which can contract and expand in the longitudinal direction LD together with the gather elastic members 631 to 633. This underpants-type disposable diaper does not have three-dimensional gathers that rise toward the front side, and has only the plane gathers of the side flaps 60. In the illustrated example, the front surface portion 61 is indirectly fixed to the back surface portion 62 via the liquid-impermeable sheet layer 64 at the fixed portion 67. However, when other members, such as the liquid-impermeable sheet layer 64, are not interposed, the front surface portion 61 is directly fixed to the back surface portion 62.

The dimension in the longitudinal direction LD of the side non-stretchable region 70 may differ or may be the same between the front end portion and the back end portion of the side flaps 60 as in the illustrated example. Note that reference numeral 60w in the figures indicates the dimension in the width direction WD of the side flaps 60. Furthermore, reference numeral w1 indicates the distance (first distance) in the width direction WD between the fixed portion 67 and the lateral edge of the side non-stretchable region 70. Reference numeral w2 indicates the dimension (second distance) in the width direction WD of a portion not fixed to the exterior body 12 in both lateral portions (side flaps) of the interior body. Reference numeral w3 indicates the dimension in the width direction WD of the fixed portion 67. Reference numeral w4 indicates the distance (third distance) in the width direction WD between the fixed portion 67 and the lateral edge of the absorbent 56. Reference numeral w5 indicates the distance (fourth distance) in the width direction WD between the fixed portion 67 and the central-side edge in the width direction WD of the front surface portion 61.

As the gather elastic members 631 to 633, elongated elastic members such as thread-shaped rubber or strip-shaped rubber can be used. When rubber threads are used, the thickness of the gather elastic members 631 to 633 is preferably in the range of 400 to 950 dtex and more preferably in the range of 470 to 780 dtex. Furthermore, the stretch rate of the gather elastic members 631 to 633 is preferably in the range of 200 to 320% and more preferably in the range of 240 to 320%. In the figures, reference numeral 631 indicates a first gather elastic member located at the first position when counted from the absorbent 56 side, reference numeral 632 indicates a second gather elastic member located at the second position when counted from the absorbent 56 side, and reference numeral 633 indicates a third gather elastic members located at the third or subsequent position when counted from the absorbent 56 side. Furthermore, reference numeral d1 indicates the interval (first interval) in the width direction WD between the lateral edge of the maximum width portion of the absorbent 56 and the first gather elastic member 631. Reference numeral d2 indicates the interval (second interval) in the width direction WD between the first gather elastic member 631 and the second gather elastic member 632. Reference numeral d3 indicates the interval (third interval) in the width direction WD between the second gather elastic member 632 and the third gather elastic member 633, as well as the interval in the width direction WD between the adjacent third gather elastic members 633.

In the side flaps 60, for bonding layers that are adjacent in the thickness direction and for fixing the gather elastic members 631 to 633 interposed therebetween, at least one of a hot melt adhesive applied by various application methods and fixation means using material welding, such as heat sealing or ultrasonic sealing, can be used. If the entire surfaces of the layers adjacent to the front side and the back side of the gather elastic members 631 to 633 are bonded, flexibility is impaired. Therefore, it is preferable that portions other than the adhered portions of the gather elastic members 631 to 633 be not adhered or weakly adhered. In the illustrated example, a configuration is provided in which a hot melt adhesive is applied only to the outer peripheral surfaces of the gather elastic members 631 to 633 by application means such as a comb gun or a SureWrap nozzle, so that the elastic members are interposed between the layers adjacent to the front and back sides, whereby the fixation of the gather elastic members 631 to 633 to the layers adjacent to the front and back sides, as well as the fixation between the layers adjacent to the front and back sides of the gather elastic members 631 to 633, is performed only by the hot melt adhesive applied to the outer peripheral surfaces of the gather elastic members 631 to 633. As in the end portions of the top sheet 30 in the illustrated example, when nonwoven fabric or sheet end portions that are separated from the gather elastic members 631 to 633 are present, a hot melt adhesive (a first hot melt adhesive HM1 in the illustrated example) can also be applied to fix them.

As the side nonwoven fabric 66, it is preferable to use one obtained by subjecting nonwoven fabric, such as spunbond nonwoven fabric (including SS, SSS, and the like), SMS nonwoven fabric (including SMS, SSMMS, and the like), or meltblown nonwoven fabric, which is flexible and has excellent uniformity and hiding performance, to water-repellent treatment using silicone or the like where necessary. It is preferable that the fiber basis weight be in the range of approximately 10 to 30 g/m². In the example illustrated in Figs. 3 and 4, as can also be understood from the fact that the front-surface nonwoven fabric layer on the base end side relative to nonwoven fabric non-present portions 65 is formed by the top sheet 30, it is possible to make the materials of the front-surface nonwoven fabric layer and the back-surface nonwoven fabric layer partially different from each other, or to make the materials different from each other.

### (Grooves)

As illustrated in Figs. 9 to 11, it is preferable that the absorbent 56 be provided with grooves 53 recessed from the front surface of the absorbent 56 to the interior thereof and having the bottom portion 51 compressed in the thickness direction. The bottom portions 51 of the grooves 53 are high-density portions that are compressed by pressing (direct pressurization) and have a substantially uniform thickness. Hereinafter, portions other than the bottom portions 51 will be referred to as non-compressed portions 52. The non-compressed portions 52 are portions that are thicker and lower in density than the bottom portions 51. However, even within the non-compressed portions 52, the density increases toward the bottom portions 51 in regions near the peripheries of the bottom portions 51 after the absorbent 56 deforms as if being pulled by the compression forming of the bottom portions 51. As long as the non-compressed portions 52 are thicker and lower in density than the bottom portions 51, the entire non-compressed portions 52 may be compressed in the thickness direction simultaneously with the compression processing for forming the bottom portions 51, or before or after the compression processing. The shape or arrangement of the non-compressed portions 52 is determined according to the shape or arrangement of the bottom portions 51.

The grooves 53 of the absorbent 56 may be formed by performing heated or unheated embossing or the like only on the absorbent 56. However, as in the absorption element 50 (i.e., together with the wrapping sheet 58) or the interior body 200, the grooves 53 may form to include the absorbent 54 and other laminated members by embossing in a state in which members other than the absorbent 56 are laminated.

When the above-described grooves 53 are provided in a continuous pattern in the width direction WD over 60% or more of the range in the longitudinal direction LD of the center region 56C and the side regions 56S, the compressed bottom portions 51 become highly dense and highly rigid, thereby improving the shape retention of the center region 56C and the side regions 56S. However, providing the compressed bottom portions 51 leads to an improvement in rigidity. Therefore, when the compressed bottom portions 51 are provided in a uniformly dense pattern over the center region 56C and the side regions 56S in consideration of the shape retention after urine absorption, the portion (the center region 56C) located at the bottom becomes less likely to bend and deform in accordance with the crotch width (reduce conformability) in a state in which the crotch portion M is deformed into a substantially U-shaped cross-sectional shape. Accordingly, in the center region 56C, it is preferable that the thickness of the bottom portions 51 of the grooves 53 be fixed, and that the area ratio thereof be lower as compared to the side regions 56S. Alternatively, although not illustrated in the figures, in the center region 56C, the area ratio of the bottom portions 51 of the grooves 53 may be fixed, while the thickness thereof may be greater as compared to the side regions 56S, or the area ratio of the bottom portions 51 of the grooves 53 may be lower, while the thickness thereof may be greater as compared to the side regions 56S. By varying at least one of the area ratio of the bottom portions 51 of the grooves 53 and the thickness of the bottom portions 51 as described above so that the rigidity of the center region 56C in the crotch portion M becomes lower than that of the side regions 56S, both the maintainability of a urine receiving space SP and conformability in the crotch, as illustrated in Fig. 8, are improved for the following reasons.
(a) The absorbent 56 becomes likely to bend at the slits 56L, with the center region 56C forming the bottom and each side region 56S rising to form the lateral wall. As a result, the crotch portion M of the diaper tends to be deformed into a substantially U-shaped cross-sectional shape, and the rigidity of the lateral walls is improved (i.e., the urine receiving space SP becomes less likely to collapse). Thus, the absorption function is also provided.
(b) Both the center region 56C and the side regions 56S are provided with the grooves 53 in a specified pattern, the grooves 53 being recessed from at least one of the front surface and the back surface of the absorption element 50 to the interior of the absorbent 56 and having the bottom portion 51 compressed in the thickness direction. Therefore, the absorbent 56 is likely to retain the shape even after urine absorption, as compared to a case in which the grooves 53 are not provided.
(c) In addition to the above advantages in (a) and (ii), the center region 56C has relatively greater flexibility, and the side regions 56S have relatively higher shape retention. Therefore, when the crotch portion M of the diaper is worn in a U-shaped cross sectional state, the lateral walls become more highly rigid and the urine receiving space SP becomes less likely to collapse, while the portion forming the bottom becomes likely to bend and deform in accordance with the crotch width.

As long as the above-described grooves 53 are provided in a continuous pattern in the width direction WD over 60% or more of the range in the longitudinal direction LD of the center region 56C and the side regions 56S, the grooves 53 may also be formed over 80% or more of the range in the longitudinal direction LD. Alternatively, as in the illustrated example, the grooves 53 may be formed over the entire range in the longitudinal direction LD. The grooves 53 may also be formed only over 60% or more and less than 100% of the range in the longitudinal direction LD from the front end or the back end of the center region 56C and the side regions 56S. Alternatively, the grooves 53 may be formed only at the intermediate portion between the front end portion and the back end portion of the center region 56C and the side regions 56S, without being formed at the front end portion and the back end portion. Particularly, when the pattern of the grooves 53 uniformly continuously extends over the entire range in the longitudinal direction LD (the entire range in the longitudinal direction LD of the absorbent 56 in the illustrated example) from a position on the front side beyond front end of the center region 56C and the side regions 56S to a position on the back side beyond the back end of the center region 56C and the side regions 56S, positioning in the longitudinal direction LD of the grooves 53 becomes unnecessary, which is preferable in terms of ease of manufacturing. However, the grooves 53 formed in the region including 60% or more of the range in the longitudinal direction LD of the center region 56C and the side regions 56S and the grooves 53 formed in the other regions may have different patterns.

The dimension, number, and the like of the bottom portions 51 of the grooves 53 can be appropriately determined. However, a depth 51h of the bottom portions 51 (a thickness 56t of the absorbent 56 minus a thickness 51t of the bottom portions of the grooves) is preferably in the range of 0.5 to 5 mm, and the area ratio of the bottom portions 51 of the grooves 53 (the ratio of the area of the bottom portions 51 to the area of the front surface of the absorption element 50) is preferably in the range of 20 to 40%.

As long as the grooves 53 having the bottom portion 51 continuously extend in the width direction WD, the grooves 53 can be provided in a continuous lattice-shaped pattern as illustrated in Figs. 10 and 11. Alternatively, although not illustrated in the figures, a plurality of grooves 53 extending along the width direction WD can be provided at intervals in the longitudinal direction LD (a horizontal stripe pattern). Alternatively, a plurality of grooves 53 extending along an oblique direction can be provided at intervals in a direction orthogonal to the continuous direction of the grooves 53 (an oblique stripe pattern). Alternatively, a plurality of grooves 53 extending in a wavy shape can be provided at intervals in the longitudinal direction LD. Alternatively, the grooves 53 can be provided in other geometric patterns or in continuous patterns having characters, animals, plants, or the like as motifs. Accordingly, the non-compressed portions 52 are intermittently arranged at least in the longitudinal direction LD. Particularly, when the grooves 53 are provided in a continuous lattice-shaped pattern, the pulp fibers and the superabsorbent polymer particles constituting the absorbent 56 are confined within unit frames 54 (minimum frames), each including the bottom portion 51 compressed in the thickness direction, which contributes to a reduction in the wrinkling or cracking of the absorbent 56.

A width 51w of the bottom portions 51 of the grooves 53 can be appropriately determined, but is generally preferably in the range of approximately 1 to 3 mm.

The thickness 51t of the bottom portions 51 of the grooves 53 can be appropriately determined, but is generally preferably in the range of 5 to 40% and particularly preferably in the range of 5 to 30% of the maximum thickness 50t of the absorption element 50.

When the grooves 53 are provided in a lattice pattern, the shape of the unit frames 54 is not particularly limited. In addition to the substantially square shape as in the illustrated example, the unit frames 54 may also have other polygonal shapes, such as a substantially diamond shape (excluding a square shape), a substantially rectangular shape, a substantially square shape, or a substantially triangular shape. Furthermore, as long as the bottom portions 51 of the grooves are provided in a lattice pattern, unit frames 54 of different shapes may also be included.

One preferred lattice-shaped pattern of the grooves 53 is, as in the example illustrated in Figs. 10 and 11, an oblique lattice-shaped pattern formed by a first portion 51a that extends in a direction inclined by 40 to 60° (more preferably 45 to 50°) clockwise in a plan view with respect to the longitudinal direction LD, and a second portion 51b that extends in a direction inclined by 40 to 60°(more preferably 45 to 50°) counterclockwise in a plan view with respect to the longitudinal direction LD. In this case, the shape of the unit frames 54 is a substantially diamond shape. It is particularly preferable that the angles of the first portion 51a and the second portion 51b with respect to the longitudinal direction LD be 45°.

The dimension of the unit frames 54 can be appropriately determined. However, if the dimension is excessively great, the rigidity improvement effect decreases. On the other hand, if the dimension is excessively small, the unit frames 54 become hard. Accordingly, it is preferable that a dimension 54x in the width direction WD of the unit frames 54 be generally in the range of approximately 15 to 20 mm. Furthermore, it is preferable that a dimension 54y in the longitudinal direction LD of the unit frames 54 be in the range of approximately 15 to 20 mm.

When the area ratio of the bottom portions 51 of the grooves 53 is to be varied, it is possible to vary the width of the bottom portions 51 of the grooves 53 while substantially fixing the pattern of the grooves 53, vary the pattern of the grooves 53 while substantially fixing the width 51w of the bottom portions 51 of the grooves 53, or vary both the pattern of the grooves 53 and the width 51w of the bottom portions 51 of the grooves 53. However, when the width 51w of the bottom portions 51 of the grooves 53 is increased in order to increase the area ratio of the bottom portions 51 of the grooves 53, the feel of the hard bottom portions 51 of the grooves 53 tends to be easily transmitted to the skin. Accordingly, as in the illustrated example, it is preferable to vary the pattern of the grooves 53 (specifically, the number of the grooves 53 per unit area) while substantially fixing the width 51w of the bottom portions 51 of the grooves 53. Furthermore, when the thickness of the bottom portions 51 of the grooves 53 is varied in the width direction WD of the absorbent 56, the degree of variation in the shape retention of the absorbent 56 is not significant, but the influence on absorption performance, such as liquid diffusibility, becomes significant, and ease of manufacturing also decreases. Accordingly, it is also possible to vary the thickness of the bottom portions 51 of the grooves 53 while substantially fixing the area ratio of the bottom portions 51 of the grooves 53. However, it is, instead, preferable to vary the area ratio of the bottom portions 51 of the grooves 53 while substantially fixing the thickness of the bottom portions 51 of the grooves 53.

Figs. 10 to 13 illustrate some preferred examples of the pattern of the grooves. In these examples, the pattern of the grooves 53 is varied in the width direction WD so that the area ratio of the bottom portions 51 of the grooves 53 in the side regions 56S becomes greater than that in the center region 56C, while the width 51w and the thickness of the bottom portions 51 of the grooves 53 are substantially fixed.

When the pattern of the grooves 53 is a lattice-shaped pattern, it is possible to make the area ratio of the bottom portions 51 of the grooves 53 in the side regions 56S higher than that in the center region 56C by combining a plurality of types of shapes of the unit frames 54 and configuring the pattern so that the areas of the unit frames 54 gradually decrease from the center toward both lateral edges in the width direction WD, as illustrated in Figs. 10, 11, and 12. Furthermore, as illustrated in Fig. 13, it is also possible to make the area ratio of the bottom portions 51 of the grooves 53 in the side regions 56S greater than that in the center region 56C by combining unit frames 54 that have the same shape (a diamond shape (square shape) in the illustrated example) but have different dimensions, including large and small dimensions, and configuring the pattern so that the areas of the unit frames 54 gradually decrease from the center toward both lateral edges in the width direction WD.

The size of the area of each unit frame 54 can be appropriately determined. However, among a pair of unit frames 54 that is adjacent in the width direction WD so as to have a common side, it is preferable that the area of the greater unit frame 54 be 1.1 to 2.5 times the area of the smaller unit frame 54. Furthermore, it is preferable that the maximum area of the unit frames 54 formed by the grooves 53 in the center region 56C be 1.5 to 4 times the maximum area of the unit frames 54 formed by the grooves 53 in the side regions 56S. In this case, it is preferable that the areas of the unit frames 54 formed by the grooves 53 in the center region 56C be in the range of 150 to 500 mm².

More specifically, the pattern of the grooves 53 in the example illustrated in Figs. 10 and 11 is an oblique lattice-shaped pattern. In this pattern, diamond-shaped first unit frames 54a having the greatest area are arranged side by side without gaps at the center in the width direction WD to form a group that continuously extends in the longitudinal direction LD. Along the lateral edges of the group of the first unit frames 54a, rectangular second unit frames 54b, each having one side common with the first unit frames 54a and a short side intersecting with the common side, the length of which is two-thirds of the length of the common side, and L-shaped third unit frames 54c, each having two sides common with the first unit frames 54a and short sides intersecting with the common sides, the length of which is two-thirds of the length of the common sides, are arranged side by side without gaps to form groups that continuously extend in the longitudinal direction LD. Along the lateral edges of the groups of the second unit frames 54b and the third unit frames 54c, diamond-shaped fourth unit frames 54d, each having one side whose length is two-thirds of the length of one side of the first unit frames 54a, are arranged side by side without gaps to form a group that continuously extends in the longitudinal direction LD. In this case, when the maximum area of the unit frames 54 of the grooves 53 is assumed to be 1, the minimum area thereof is 4/9. Although this variation is smaller compared to the two examples that will be described later, it is preferable in terms of ease of manufacturing.

Furthermore, the pattern of the grooves 53 in the example illustrated in Fig. 12 is also an oblique lattice-shaped pattern. In this pattern, diamond-shaped first unit frames 54e having the greatest area are arranged side by side without gaps at the center in the width direction WD to form a group that continuously extends in the longitudinal direction LD. Along the lateral edges of the group of the first unit frames 54e, diamond-shaped second unit frames 54f, each having one side whose length is one-half of the length of one side of the first unit frames 54e, and L-shaped third unit frames 54g, each having two long sides whose length is equal to the length of one side of the first unit frames 54e and four short sides whose length is one-half of the length of one side of the first unit frames 54e, are arranged side by side without gaps to form groups that continuously extend in the longitudinal direction LD. Along the lateral edges of the groups of the third unit frames 54g, the diamond-shaped second unit frames 54f, each having one side whose length is one-half of the length of one side of the first unit frames 54e, are arranged side by side without gaps to form groups that continuously extend in the longitudinal direction LD. In this case, when the maximum area of the unit frames 54e to 54g is assumed to be 1, the minimum area thereof is 1/4.

Furthermore, the pattern of the grooves 53 in the example illustrated in Fig. 13 is also an oblique lattice-shaped pattern. In this pattern, diamond-shaped first unit frames 54h having the greatest area are arranged side by side without gaps at the center in the width direction WD to form a group that continuously extends in the longitudinal direction LD. Along the lateral edges of the group of the first unit frames 54h, diamond-shaped second unit frames 54i, each having one side whose length is one-half of the length of one side of the first unit frames 54h, are arranged side by side without gaps to form a group that continuously extends in the longitudinal direction. Along the lateral edges of the groups of the second unit frames 54i, diamond-shaped third unit frames 54j, each having one side whose length is one-third of the length of one side of the first unit frames 54h, are arranged side by side without gaps to form a group that continuously extends in the longitudinal direction LD. In this case, when the maximum area of the unit frames 54h to 54j is assumed to be 1, the minimum area thereof is 1/9.

When the pattern of the grooves 53 is a lattice-shaped pattern, as in the example illustrated in Fig. 10, at least one unit frame 54 is present in the side regions 56S if the dimension in the width direction WD of the unit frames 54 in the side regions 56S is 0.5 times or less and preferably 0.3 to 0.5 times the minimum dimension in the width direction WD of the side regions 56S. Even if the position of the lattice-shaped pattern slightly deviates due to manufacturing errors, the configuration is particularly preferable in terms of both rigidity improvement and diffusibility improvement.

In this case, as in the example illustrated in Fig. 10, the pattern of the grooves 53 in both the side regions 56S is formed only by slit-adjacent frames 54m, each formed by a lateral edge of a slit 56L and a groove 53, lateral-edge-adjacent frames 54n, each formed by a lateral edge 56e of the absorbent 56 and a groove 53, and complete unit frames 54 (without omissions), each having the same dimension and the same shape. When the area of each slit-adjacent frame 54m and the area of each lateral-edge-adjacent frame 54n are smaller than the area of the unit frames 54, the rigidity of the lateral walls becomes particularly high when the diaper deforms into a substantially U-shaped cross-sectional shape, which is preferable because the urine receiving space SP becomes less likely to collapse.

Of course, in the side regions 56S, as in the example illustrated in Fig. 12 or the example illustrated in Fig. 13, the area of some or all of the slit-adjacent frames 54m (the same applies to the lateral-edge-adjacent frames 54n) may be greater than the area of the unit frames 54.

On the other hand, as in the illustrated example, it is preferable that the pattern of the grooves 53 in the center region 56C have the complete unit frames 54 (without omissions), each having the same dimension and the same shape. In this case, the slit-adjacent frames 54m, each formed by a lateral edge of a slit 56L and a groove 53 as in the illustrated example, may also be provided.

Although not illustrated in the figures, by aligning the direction and position of the lateral edges of the slits 56L with the direction and position of the grooves 53, the pattern of the grooves 53 in at least one of the side regions 56S and the center region 56C can be formed only by the complete unit frames 54 (without omissions).

On the other hand, the shapes of the absorbent 56 and the slits 56L have been described above. However, as illustrated in Fig. 14(a), when both lateral edges of the absorbent 56 extend linearly along the longitudinal direction LD, and the pair of slits 56L extend so as to approach the lateral edges 56e of the absorbent 56 from an intermediate position in the longitudinal direction LD toward the front side and the back side, respectively, there are advantages that, in a state in which the crotch portion M of the diaper is deformed into a substantially U-shaped cross-sectional shape, the lateral walls easily fit to the inner sides of the bases of the thighs, and the lateral walls are lowered toward both sides in the longitudinal direction and thus become less likely to bend. As a result, in that vicinity, the urine receiving space SP becomes particularly less likely to collapse.

### <Explanation of Terms Used in Specification>

The following terms used in the specification shall have the following meanings unless otherwise specifically stated in the specification.
·The term "longitudinal direction" refers to the direction (vertical direction) indicated by reference numeral LD in the figures, the term "width direction" refers to the direction (right-left direction) indicated by WD in the figures, and the longitudinal direction and the width direction are orthogonal to each other.
·The term "surface side" refers to the side that is closer to the skin of the wearer when worn, and the term "back side" refers to the side that is farther from the skin of the wearer when worn.
·The term "front surface" refers to the surface that is closer to the skin of the wearer when worn, and the term "back surface" refers to the surface that is farther from the skin of the wearer when worn.
·The term "stretch rate" refers to a value based on the assumption that the natural length is 100%. For example, an stretch rate of 200% is equivalent to a stretch magnification of two times.
·The "gel strength" is measured in the following manner. 1.0 g of superabsorbent polymer is added to 49.0 g of artificial urine (a mixture of 2 wt% urea, 0.8 wt% sodium chloride, 0.03 wt% calcium chloride dihydrate, 0.08 wt% magnesium sulfate heptahydrate, and 97.09 wt% ion-exchanged water), and the mixture is stirred using a stirrer. The generated gel is left to stand for three hours in a constant temperature and humidity chamber at 40°C and 60% RH, and then returned to room temperature. The gel strength is measured using a curdmeter (Curdmeter-MAX ME-500 manufactured by I. techno Engineering Co., Ltd).
·The "basis weight" is measured in the following manner. After preliminary drying, a sample or specimen is left to stand in a testing room or apparatus under standard conditions (the testing place has a temperature of 23 ± 1°C and a relative humidity of 50 ± 2%) until it has a constant weight. The preliminary drying refers to bringing a sample or specimen to a constant weight at 100°C. Note that fibers having an official moisture regain of 0.0% may not undergo the preliminary drying. From the specimen that has reached a constant weight, a 100 mm × 100 mm sample is cut off using a sample collecting mold (100 mm × 100 mm). The weight of the sample is measured, multiplied by 100 to calculate the weight per square meter, and used as the basis weight.
·The "thickness" of thick members, such as the absorbent 56, the absorption element 50, and the bottom portions 51 of the grooves, is measured using a thickness measurement device manufactured by OZAKI MFG. CO., Ltd. (PEACOCK, dial thickness gauge, Type H (with a measurement range of 0 to 10 mm, a circular terminal having a measurement area diameter of 10 mm, a measuring force of approximately 1.7 N, and a pressure of approximately 21.7 KPa)). The measurement is performed with a sample and the thickness measurement device put in a horizontal position.
·The "thickness" of thin sheets, such as nonwoven fabric, is automatically measured using an automatic thickness measurement device (KES-G5, handy compression measurement program) under conditions of a load of 0.098 N/cm2 and a pressed area of 2 cm².
·The water absorption capacity is measured in accordance with JIS K7223-1996, "Testing Method for Water Absorption Capacity of Superabsorbent Polymers."
·The water absorption rate is defined as the "time until the endpoint" when the test in JIS K7224-1996, "Testing Method for Water Absorption Rate of Superabsorbent Polymers," is conducted using 2 g of superabsorbent polymer and 50 g of physiological saline.
·The "developed state" refers to a state that is expanded to be flat without contraction (including all types of contraction, such as contraction caused by elastic members) or looseness.
·The "maximum expansion" refers to the maximum expansion value in the stretchable direction of the stretchable region (i.e., the expansion at the elastic limit, which is equivalent to expansion in the developed state), and the length in the developed state is expressed as percentage when the natural length is assumed to be 100%.
·Unless otherwise specifically stated, the dimension of each portion refers to the dimension in the developed state, not in the natural-length state.
·When no environmental conditions for testing or measurement are specified, the testing or measurement shall be conducted in a testing room or apparatus under standard conditions (the testing place has a temperature of 23 ± 1°C and a relative humidity of 50 ± 2%).

### Industrial Applicability

The present invention can be applied not only to the underpants-type disposable diaper as in the above example, but also to all types of diapers, such as tape-type disposable diapers and pad-type disposable diapers.

### Reference Signs List

- 11: Liquid-impermeable sheet
- 12A: Side seal
- 12B: Back exterior body
- 12F: Front exterior body
- 12H: Inner sheet layer
- 12S: Outer sheet layer
- 12X: Non-stretchable region
- 13: Cover nonwoven fabric layer
- 14: Gluteal cover portion
- 15: Underwaist elastic member
- 16: Gluteal cover elastic member
- 17: Waist elastic member
- 200: Interior body
- 201: Fixed region
- 30: Top sheet
- 50: Absorption element
- 51: Bottom portion
- 52: Non-compressed portion
- 53: Groove
- 54: Unit frame
- 54m: Slit-adjacent frame
- 54n: Lateral-edge-adjacent frame
- 56: Absorbent
- 56C: Center region
- 56L: Slit
- 56S: Side region
- 58: Wrapping sheet
- 60: Side flap
- 61: Front surface portion
- 62: Back surface portion
- 631 to 633: Gather elastic member
- 631: First gather elastic member
- 632: Second gather elastic member
- 633: Third gather elastic member
- 64: Liquid-impermeable sheet layer
- 65: Nonwoven fabric non-present portion
- 66: Side nonwoven fabric
- 66r: Folded-back portion
- 67: Fixed portion
- 70: Side non-stretchable region
- 80: Plane gather region
- A1: Intermittent stretchable area
- A2: Continuous stretchable area
- B: Back body
- BT: Back lower torso portion
- F: Front body
- FT: Front lower torso portion
- HM1: First hot melt adhesive
- HM2: Second hot melt adhesive
- HM3: Third hot melt adhesive
- L: Intermediate region
- LD: Longitudinal direction
- M: Crotch portion
- SP: Urine receiving space
- T: Lower torso region
- U: Lower waist portion
- W: Waist portion
- WD: Width direction
- WO: Waist opening
- d1: First interval
- d2: Second interval
- d3: Third interval
- w1: First distance
- w2: Second distance
- w3: Dimension in width direction of fixed portion
- w4: Third distance
- w5: Fourth distance

## Claims

1. A disposable diaper comprising:
a crotch portion;
a front portion and a back portion extending to a front side and a back side of the crotch portion, respectively; and
an absorption element having an absorbent provided over a range in a longitudinal direction including the crotch portion, and a wrapping sheet wrapping the absorbent, wherein
the absorbent is formed by mixing and accumulating pulp fibers and superabsorbent polymer particles,
the absorbent has a pair of elongated slits extending in the longitudinal direction, the slits being provided on one side and the other side in a width direction of the crotch portion, respectively,
the absorbent has a center region defined between the one slit and the other slit, and a pair of side regions defined between the slits on both sides in the width direction and lateral edges of the absorbent,
grooves are provided in the center region and the side regions in a continuous pattern in the width direction over 60% or more of a range thereof in the longitudinal direction, the grooves being recessed from at least one of a front surface and a back surface of the absorbent to an interior of the absorbent and having a bottom portion compressed in a thickness direction, and
the center region has, as compared to the side regions, a fixed thickness in the bottom portions of the grooves and a lower area ratio in the bottom portions of the grooves, a fixed area ratio in the bottom portions of the grooves and a greater thickness in the bottom portions of the grooves, or a lower area ratio in the bottom portions of the grooves and a greater thickness in the bottom portions of the grooves.

2. The disposable diaper according to claim 1, wherein
the center region and the side regions are each regions in which the grooves continuously extend in a lattice-shaped pattern,
a thickness and a width of the bottom portions of the grooves are the same in the center region and the side regions, and
a maximum area of unit frames formed by the grooves in the center region is 1.5 to 4 times a maximum area of unit frames formed by the grooves in the side regions.

3. The disposable diaper according to claim 2, wherein
the absorbent is composed of one or a plurality of layers formed by mixing and accumulating pulp fibers and superabsorbent polymer particles,
a basis weight of the pulp fibers in the absorbent is in a range of 80 to 450 g/m²,
a weight ratio of the pulp fibers to the superabsorbent polymer particles in the absorbent is in a range of 20:80 to 80:20,
a maximum thickness of the absorbent is in a range of 1 to 20 mm,
the thickness of the bottom portions is 5 to 40% of the maximum thickness of the absorbent,
the width of the bottom portions is in a range of 1 to 3 mm, and
the area of the unit frames formed by the grooves in the center region is in a range of 150 to 500 mm².

4. The disposable diaper according to claim 2 or 3, wherein
the grooves are formed in an oblique lattice-shaped pattern formed by a first portion that is inclined by 40 to 60°clockwise in a plan view with respect to the longitudinal direction, and a second portion that is inclined by 40 to 60° counterclockwise in a plan view with respect to the longitudinal direction.

5. The disposable diaper according to claim 2 or 3, wherein
a dimension in the width direction of the unit frames in the side regions is 0.5 times or less a minimum dimension in the width direction of the side regions.

6. The disposable diaper according to claim 5, wherein
a pattern of the grooves in both the side regions is formed only by slit-adjacent frames formed by lateral edges of the slits and the grooves, lateral-edge-adjacent frames formed by lateral edges of the absorbent and the grooves, and the complete unit frames that have a same dimension and a same shape, and
an area of each slit-adjacent frame and an area of each lateral-edge-adjacent frame in both the side regions are smaller than an area of the unit frames.

7. The disposable diaper according to claim 1 or 2, wherein
both the lateral edges of the absorbent extend linearly along the longitudinal direction, and
the pair of slits extends so as to approach the lateral edges of the absorbent from an intermediate position thereof in the longitudinal direction toward the front side and the back side.
